(19) 

Europäisches
Patentamt
European
Patent Office
Office européen
des brevets

(11) **EP 3 226 163 A1**

(12) **EUROPEAN PATENT APPLICATION**
published in accordance with Art. 153(4) EPC

(43) Date of publication:
**04.10.2017 Bulletin 2017/40**

(21) Application number: **15862302.5**

(22) Date of filing: **25.11.2015**

(51) Int Cl.:
***G06F 19/18*** *(2011.01)*

(86) International application number:
**PCT/JP2015/083068**

(87) International publication number:
**WO 2016/084844 (02.06.2016 Gazette 2016/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.11.2014 JP 2014238252**

(71) Applicant: **Iwate Medical University Educational
Foundation
Morioka-shi, Iwate 020-8505 (JP)**

(72) Inventor: **HACHIYA, Tsuyoshi
Morioka-shi
Iwate 020-8505 (JP)**

(74) Representative: **J A Kemp
14 South Square
Gray's Inn
London WC1R 5JJ (GB)**

(54) **TRAIT PREDICTION MODEL CREATION METHOD AND TRAIT PREDICTION METHOD**

(57) To provide methods of creating trait prediction models for predicting phenotypes of traits from single nucleotide polymorphism data and methods of predicting traits with which traits can be predicted with a high accuracy.

This is a method of creating a trait prediction model for predicting a phenotype of a multifactorial trait using data of a plurality of single nucleotide polymorphisms linked to a trait for each of a plurality of individuals of an organism: representing each of the plurality of single nucleotide polymorphisms as a matrix; classifying the plurality of single nucleotide polymorphisms into a plurality of categories based on their genetic architectures; calculating, for each of the categories, a genomic similarity matrix using the represented matrix and the number of the single nucleotide polymorphisms belonging to the category; and applying the genomic similarity matrix and a parameter of the genetic architecture to a linear mixed model.

**EP 3 226 163 A1**

**Description**

Cross reference to related document

The present application claims the priority of Japanese Patent Application No. 2014-238252 filed November 25, 2014, which is incorporated herein by reference.

Technical Field

The present invention relates to methods of creating trait prediction models and methods of predicting traits.

Background Art

For phenotypic prediction using human genomic information, methods of predicting a phenotype using only a susceptibility polymorphism already identified have mainly been investigated, focusing on trait susceptibility polymorphisms (see, V. Lyssenko et al., N Engl J Med 2008 vol. 359 p. 2220-2232; S. Ripatthi et al., Lanet 2010 Vol. 376 p. 1393-1400; C. A. Ibrahim-Verbaas et al., Stroke 2014 vol. 45 p. 403-412). These methods enumerate several hundred polymorphisms related to traits and estimate a weight of each polymorphism; they are thus easy to be intuitively understood since effects of individual polymorphisms on traits can be expressed numerically.

The sole use of the susceptibility polymorphisms is, however, a disadvantage and the limit of this approach. This is because in almost all multifactorial traits, only a few of the susceptibility polymorphisms that are actually responsible have been identified. For example, it is estimated that about 80% of the variance in body height can be explained by genetic factors, but the variance explained by a known susceptibility polymorphism is only about 5%.

With this respect, non-patent literature document (D. Speed and D. J. Balding, Genome Research 2015 vol. 24 p. 1550-1557) discloses a method of predicting phenotypes using exhaustive (genome-wide) polymorphism information regardless of susceptibility polymorphisms. Specifically, a plurality of single nucleotide polymorphisms (SNPs) are divided into a plurality of categories, and a linear mixed model is applied thereto. The accuracy of prediction of the method is, however, still insufficient.

Summary of Invention

Technical Problem

An object of the present invention is to provide methods of creating trait prediction models for predicting phenotypes of traits from single nucleotide polymorphism data and methods of predicting traits with which traits can be predicted with a high accuracy.

Solution to Problem

The present inventors have investigated a statistical processing method using exhaustive (i.e. genome-wide) polymorphism information regardless of susceptibility polymorphisms. Specifically, taking 27 qualitative traits including the body height and HbA1c value and 5 qualitative traits including diseases of diabetes and low HDL cholesterolemia as examples, the present inventors utilized a linear mixed model using about 1 million polymorphisms as genomic information and gender/age information as adjustment variables and trained the model about the traits to create a prediction model. The present inventors found that this prediction was highly correlated with measured values, and thus accomplished a method of predicting phenotypes from genomic information.

An aspect of the present invention is a method of creating a trait prediction model for predicting a phenotype of a multifactorial trait using data of a plurality of single nucleotide polymorphisms linked to a trait for each of a plurality of individuals of an organism, the method including the steps of: representing each of the plurality of single nucleotide polymorphisms as a matrix; classifying the plurality of single nucleotide polymorphisms into a plurality of categories based on their genetic architectures; calculating, for each of the categories, a genomic similarity matrix using the represented matrix and the number of the single nucleotide polymorphisms belonging to the category; and applying the genomic similarity matrix and a parameter of the genetic architecture to a linear mixed model. The genetic architecture may be an effect size and/or an allele frequency.

Another aspect of the present invention is a method of creating a trait prediction model for predicting a phenotype of a multifactorial trait using data of gender, age and a plurality of single nucleotide polymorphisms linked to a trait for each of a plurality of individuals of an organism, the method including the steps of: representing each of the plurality of single nucleotide polymorphisms as a matrix; representing the gender and/or age as a matrix; calculating a genomic similarity matrix using the represented matrix of the single nucleotide polymorphisms and the number of the single nucleotide polymorphisms; and applying the genomic similarity matrix and the matrix of the gender and/or age to a linear mixed model. The trait may be selected from the group consisting of the body height, body weight, systolic blood pressure, diastolic blood pressure, blood glucose, HbA1c, red blood cell number, hemoglobin, corpuscular volume, white blood cell number, platelet number, percentage of neutrophils, percentage of lymphocytes, percentage of monocytes, percentage of eosinophils, percentage of basophils, percentage of large unstained cells, AST (GOT), ALT (GPT), γ-GTP, total cholesterol, neutral fat, HDL cholesterol, LDL cholesterol, creatinine, urea nitrogen, uric acid, diabetes, hypertension, high LDL cholesterolemia, low HDL cholesterolemia, and hypertriglyceridemia.

A further aspect of the present invention is a method of predicting a trait of an individual of an organism from a plurality of single nucleotide polymorphism data in the individual of the organism, including the steps of: creating a prediction model using a set of training data according to the aforementioned method of creating a trait prediction model; determining a parameter and a hidden variable of a linear mixed model; and applying the plurality of single nucleotide polymorphism data of the individual of the organism to the prediction model.

A yet further aspect of the present invention is a program for predicting a trait of an individual of an organism from a plurality of single nucleotide polymorphism data in the individual of the organism, by which the computer is caused to execute the aforementioned method of predicting a trait. An aspect of the present invention may be a computer readable recording medium in which the present program has been recorded.

A further aspect of the present invention is a trait prediction system for predicting a trait of an individual of an organism from a plurality of single nucleotide polymorphism data, including: (i) an input device for inputting a plurality of single nucleotide polymorphism data of the individual of the organism; (ii) a computer that executes the above program using data that has been input, and (iii) an output device for outputting the result obtained in (ii).

Brief description of drawings

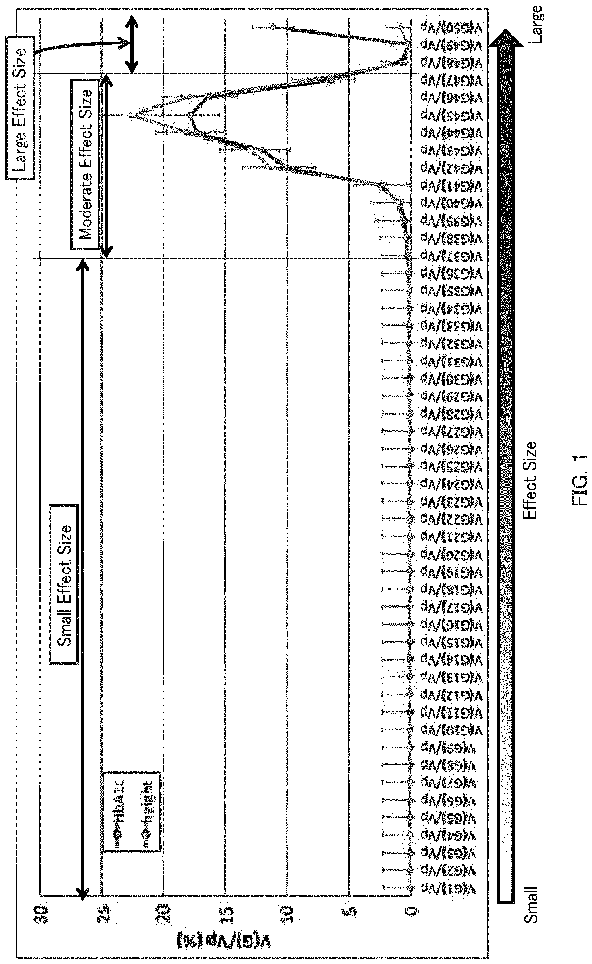

[Fig. 1] Figure 1 represents a diagram showing estimated contribution ratios (with $Q_{es}$ = 50 and $Q_{RAF}$ = 1) obtained by a genetic architecture division method, focusing on HbA1c values and body heights, in an example of the present invention.

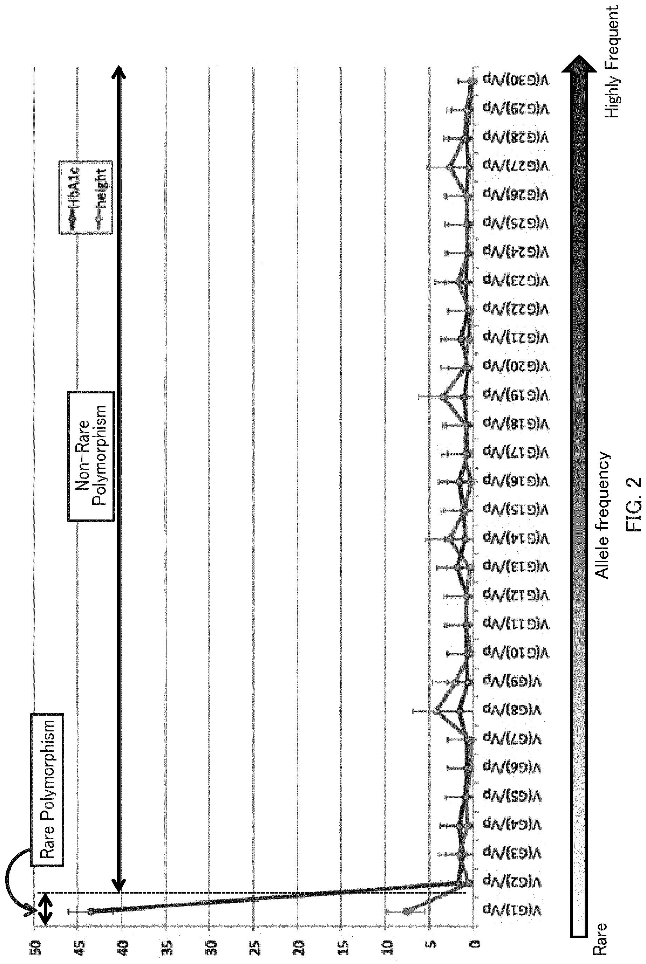

[Fig. 2] Figure 2 represents a diagram showing estimated contribution ratios (with $Q_{es}$ = 1 and $Q_{RAF}$ = 30) obtained by a genetic architecture division method, focusing on HbA1c values and body heights in an example of the present invention.

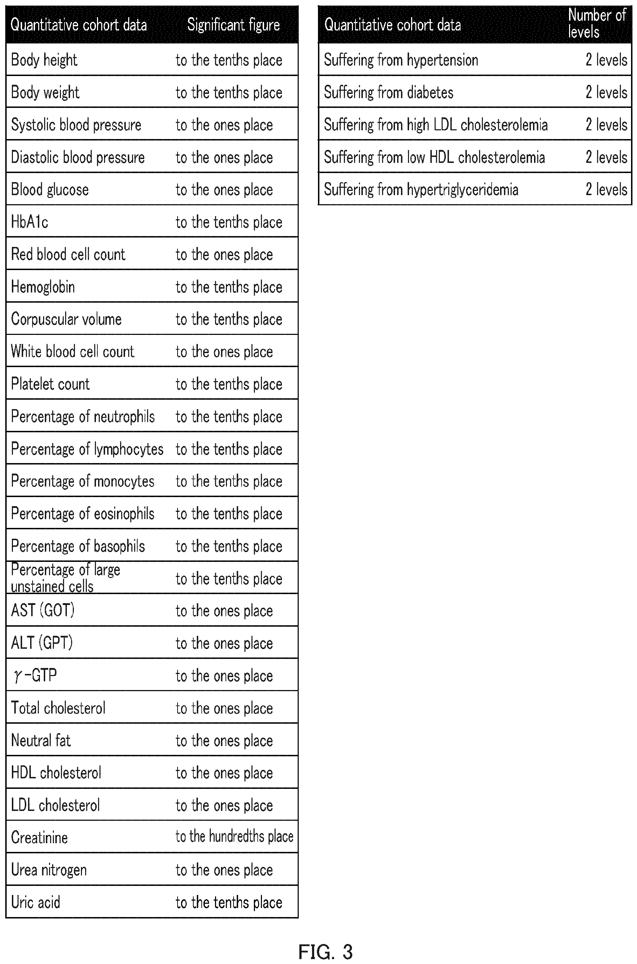

[Fig. 3] Figure 3 represents a list of traits used in examples of the present invention.

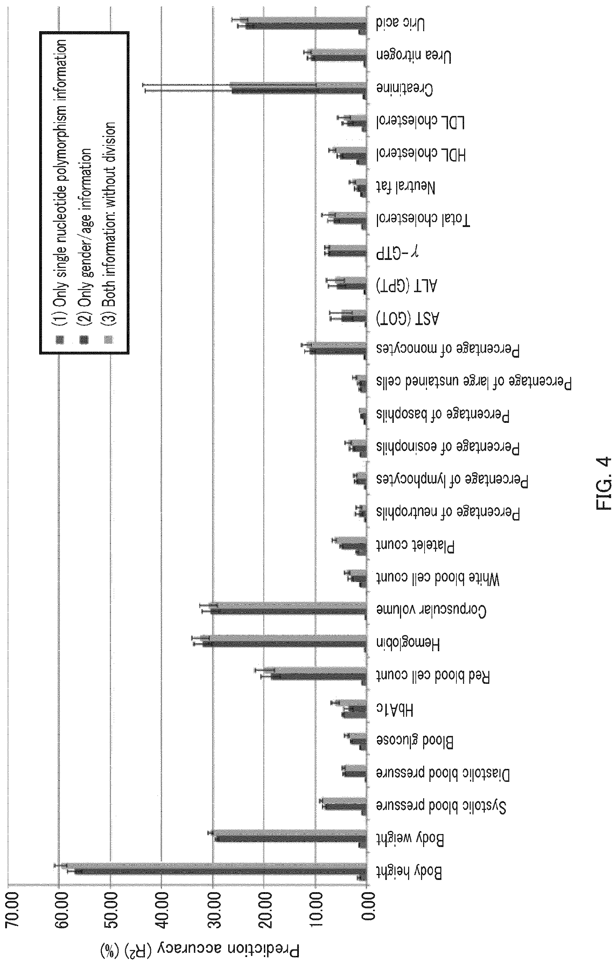

[Fig. 4] Figure 4 represents a diagram showing results of accuracy evaluation for 27 quantitative traits in an example of the present invention. The following three cases were compared: (1) only the single nucleotide polymorphism information was used and $Q_{es}$ = 1 and $Q_{RAF}$ = 1 (without the genetic architecture division); (2) only the gender/age information was used; and (3) both the single nucleotide polymorphism information and the gender/age information were used and $Q_{es}$ = 1 and $Q_{RAF}$ = 1 (without the genetic architecture division; the examples of the present invention). A coefficient of determination $R^2$ between measured and predicted values (i.e., a squared correlation coefficient) was used as an evaluation measure and the evaluation was performed using a 2-fold cross validation method.

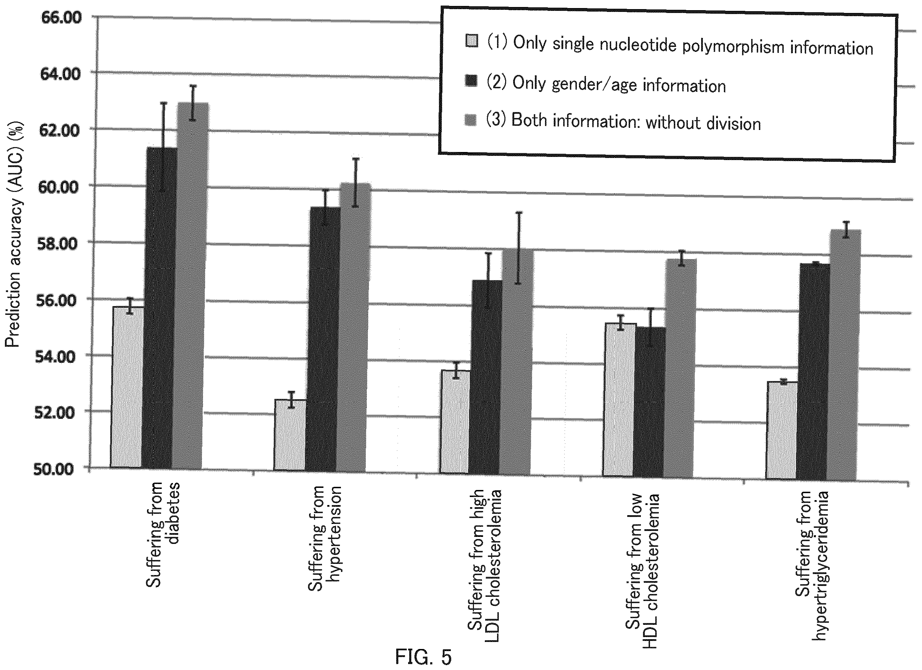

[Fig. 5] Figure 5 represents a diagram showing results of accuracy evaluation for 5 qualitative traits in an example of the present invention. The following three cases were compared: (1) only the single nucleotide polymorphism information was used and $Q_{es}$ = 1 and $Q_{RAF}$ = 1 (without the genetic architecture division); (2) only the gender/age information was used; and (3) both the single nucleotide polymorphism information and the gender/age information were used and $Q_{es}$ = 1 and $Q_{RAF}$ = 1 (without the genetic architecture division; the examples of the present invention). AUC was used as an evaluation measure and the evaluation was performed using a 2-fold cross validation method.

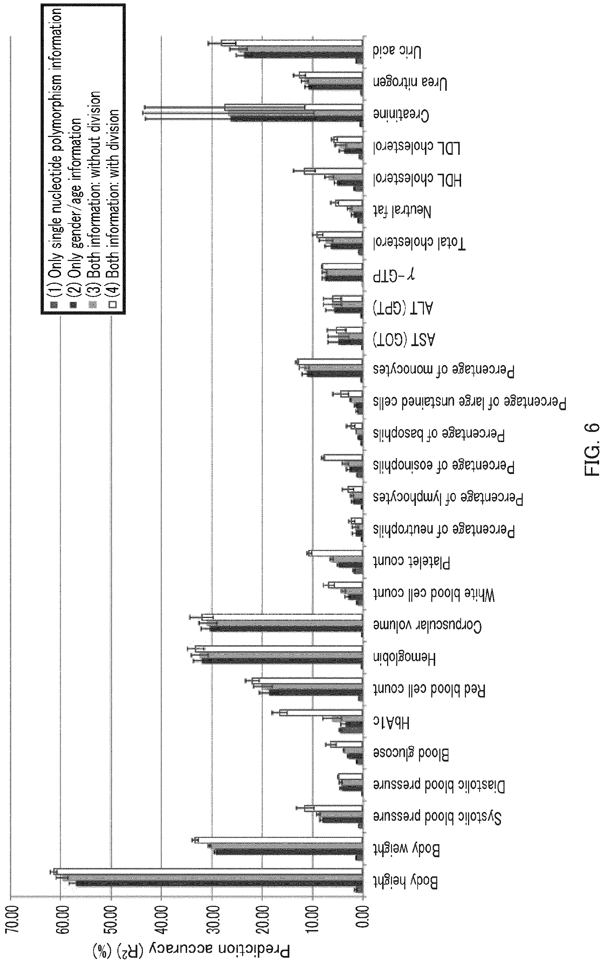

[Fig. 6] Figure 6 represents a diagram showing results of accuracy evaluation for 27 quantitative traits with sufficient amount of samples in an example of the present invention. The following four methods were compared: (1) only the single nucleotide polymorphism information was used and $Q_{es}$ = 1 and $Q_{RAF}$ = 1 (without the genetic architecture division); (2) only the gender/age information was used; (3) both the single nucleotide polymorphism information and the gender/age information were used and $Q_{es}$ = 1 and $Q_{RAF}$ = 1 (without the genetic architecture division; the examples of the present invention); and (4) both the single nucleotide polymorphism information and the gender/age information were used and $Q_{es}$ = 10 and $Q_{RAF}$ = 1 (with the genetic architecture division; the examples of the present invention). A coefficient of determination $R^2$ between measured and predicted values (i.e., a squared correlation coefficient) was used as an evaluation measure and the evaluation was performed using a 2-fold cross validation method.

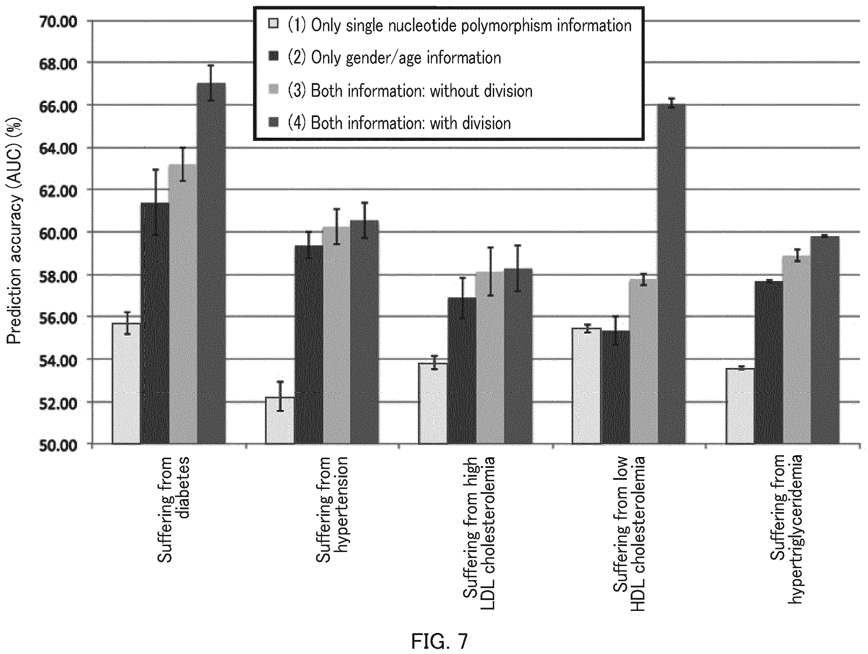

[Fig. 7] Figure 7 represents a diagram showing results of accuracy evaluation for 5 qualitative traits with sufficient amount of samples in an example of the present invention. The following four methods were compared: (1) only the single nucleotide polymorphism information was used and $Q_{es}$ = 1 and $Q_{RAF}$ = 1 (without the genetic architecture division); (2) only the gender/age information was used; (3) both the single nucleotide polymorphism information and the gender/age information were used and $Q_{es}$ = 1 and $Q_{RAF}$ = 1 (without the genetic architecture division; the examples of the present invention); and (4) both the single nucleotide polymorphism information and the gender/age information were used and $Q_{es}$ = 10 and $Q_{RAF}$ = 1 (with the genetic architecture division; the examples of the present invention). AUC was used as an evaluation measure and the evaluation was performed using a 2-fold cross validation method.

Description of Embodiments

The objects, features, advantages, and ideas of the present invention are apparent to those skilled in the art from the description of this specification. Furthermore, those skilled in the art can easily reproduce the present invention from the description herein. The embodiments and specific examples described below represent preferable embodiments of the present invention, which are given for the purpose of illustration or explanation. The present invention is not limited thereto. It is obvious to those skilled in the art that various changes and modifications may be made according to the description of the present specification within the spirit and scope of the present invention disclosed herein.

A method of creating a trait prediction model according to the present invention is a method of creating a trait prediction model for predicting a phenotype of a multifactorial trait using data of a plurality of single nucleotide polymorphisms linked to a trait for each of a plurality of individuals of an organism, the method including the steps of: representing each of the plurality of single nucleotide polymorphisms as a matrix; classifying the plurality of single nucleotide polymorphisms into a plurality of categories based on their genetic architectures; calculating, for each of the categories, a genomic similarity matrix using the represented matrix of the single nucleotide polymorphisms and the number of the single nucleotide polymorphisms belonging to each category; and applying the genomic similarity matrix and a parameter of the genetic architecture to a linear mixed model; or a method of creating a trait prediction model for predicting a phenotype of a multifactorial trait using data of gender, age and a plurality of single nucleotide polymorphisms linked to a trait for each of a plurality of individuals of an organism, the method including the steps of: representing each of the plurality of single nucleotide polymorphisms as a matrix; representing the gender and/or age as a matrix; calculating a genomic similarity matrix using the represented matrix of the single nucleotide polymorphisms and the number of the single nucleotide polymorphisms; and applying the genomic similarity matrix and the matrix of the gender and/or age to a linear mixed model.

The single nucleotide polymorphisms contained in the single nucleotide polymorphism data used here are not particularly limited and may or may not be a susceptibility polymorphism on a target trait. The number and type of the single nucleotide polymorphisms to be used are also not particularly limited, but it is preferable to encompass all single nucleotide polymorphisms that occur at a frequency of at least 1% in a population of individuals of a target organism.

The target organism is not particularly limited, and it may be a plant or an animal, but the target organism is preferably a vertebrate, more preferably a mammal, and most preferably human. The target trait is not particularly limited as long as it is a multifactorial trait, and for example, in the case of human, examples of the traits include indexes relating to the body such as the body height, body weight and BMI; blood test values such as blood pressure (i.e., systolic blood pressure and/or diastolic blood pressure), HbA1c, red blood cell number, hemoglobin, corpuscular volume, white blood cell number, platelet number, percentage of neutrophils, percentage of lymphocytes, percentage of monocytes, percentage of eosinophils, percentage of basophils, percentage of large unstained cells, percentage of nucleated red blood cells, AST (GOT), ALT (GPT), $\gamma$-GTP, total cholesterol, neutral fat, HDL cholesterol, LDL cholesterol, creatinine, urea nitrogen, estimated glomerular filtration rate, and uric acid; abilities such as memory, understanding, intelligence index, and exercise skill; and susceptibility to diseases such as lifestyle related diseases including obesity, diabetes, hypertension, and cardiovascular disease, cancer, and immunity diseases including allergy and autoimmune diseases.

By using the method of creating a prediction model of the present invention, it is possible to predict a trait of an individual of an organism from a plurality of single nucleotide polymorphism data. More specifically, a trait prediction model is created and parameters and hidden variables of the linear mixed model are determined using a set of training data according to the method of creating a trait prediction model of the present invention; and then a plurality of single nucleotide polymorphism data are applied to the trait prediction model, thereby it is possible to predict traits of the individual of the organism.

Hereinafter, methods of creating a prediction model and methods of predicting traits of the present invention will be described in detail and specifically with referring to examples, but the present invention is not limited to these embodiments or examples.

(1) Matrix representation of gender/age information

Given that gender and age data have already been obtained for $N$ human individuals, a process of representing these data as an *N*-by-6 matrix *X* is described. Each row vector of the matrix *X* represents the gender/age information of the corresponding individual. An element in the *i*-th row and *j*-th column of the matrix *X* is herein denoted as *X(i,j)*. Age is treated as categorical data, but the number of categories is not particularly limited. Here, described is an example where the following five categories are used: age 39 or younger, age 40 to 49, age 50 to 59, age 60 to 69, and age 70 or over.

The gender information is arranged at the first column of the matrix *X*. When the *i*-th human individual is given a gender designation "*M*" for male and "*F*" for female, an element *X(i,1)* is defined by:

$$X(i,1) = \begin{cases} 0 & for\ "F" \\ 1 & for\ "M". \end{cases}$$

The age information is arranged at the columns 2 to 6 of the matrix *X*. When the age of the *i*-th human individual is $age_i$, elements *X(i,2)*, *X(i,3)*, *X(i,4)*, *X(i,5)*, and *X(i,6)* are defined by:

$$X(i,2) = \begin{cases} 1 & age_i \leq 39 \\ 0 & otherwise \end{cases}$$

$$X(i,3) = \begin{cases} 1 & 40 \leq age_i \leq 49 \\ 0 & otherwise \end{cases}$$

$$X(i,4) = \begin{cases} 1 & 50 \leq age_i \leq 59 \\ 0 & otherwise \end{cases}$$

$$X(i,5) = \begin{cases} 1 & 60 \leq age_i \leq 69 \\ 0 & otherwise \end{cases}$$

$$X(i,6) = \begin{cases} 1 & 70 \leq age_i \\ 0 & otherwise. \end{cases}$$

(2) Matrix representation of genomic information

Given that *p* single nucleotide polymorphism (SNP) data have already been obtained for *N* human individuals, a process of representing these data as an *N*-by-*p* matrix *W* (where *N* and *p* are each an integer of 1 or larger) is described. Each row vector of the matrix *W* represents a polymorphism profile in the corresponding individual and each column vector of the matrix *W* represents a vector indicating differences between or among individuals for a certain polymorphism site.

The *j*-th polymorphism of the *i*-th human individual has two alleles. An individual with both alleles identical to the human representative sequence is denoted as "AA", a human with only one allele identical to the human representative sequence is denoted as "AB", and a human with both alleles not identical to the human representative sequence is denoted as "BB". The element in the *i*-th row and *j*-th column of the matrix *W* is denoted as *W(i,j)*. The allele frequency of the *j*-th polymorphism is denoted *as $f_j$*. With these denotations, an element *W(i,j)* is defined by:

$$W(i,j) = \begin{cases} \dfrac{-2f_j}{\sqrt{2f_j(1-f_j)}} & for\ "AA" \\[2ex] \dfrac{1-2f_j}{\sqrt{2f_j(1-f_j)}} & for\ "AB" \\[2ex] \dfrac{2-2f_j}{\sqrt{2f_j(1-f_j)}} & for\ "BB". \end{cases}$$

The representative sequence herein is a sequence having nucleotides determined for respective polymorphisms, but it may be, for example, a publicly-available sequence that has been obtained in a genome project.

(3) Classification of SNPs based on genetic architectures

A way of classifying *p* SNPs into multiple categories based on their genetic architectures is described below. Specific parameters of genetic architecture include an effect size, which is a parameter of the strength of the relationship with a trait, and an allele frequency, which represents the frequency of SNPs in a human population. Representative specific examples of the effect size include relative risk, odds ratio, coefficient of determination, and regression coefficient. Examples of the allele frequency include risk allele frequency (RAF) and minor allele frequency (MAF). Although the parameters describing the genetic architecture used in the method of the present invention are not specifically limited, a classification process with the regression coefficient and RAF is shown as an example.

(4) Division procedure (1): calculation of $Q_{es}$ quantiles for effect sizes

For a positive integer $Q_{es}$, ($Q_{es}$ - 1) values dividing the distribution into $Q_{es}$ equal parts are calculated. A specific method of calculating quantiles is shown below, but the method of calculating the quantiles is not limited thereto. When the data obtained by sorting the effect sizes of the SNPs in ascending order is $es_1 \le es_2 \le ... \le es_p$, the *i*-th $Q_{es}$-quantile

$Q_{es}^{(i)}$ $(1 \le i \le Q_{es} - 1)$ is given by:

$$m_i = \frac{i \times p}{Q_{es}}$$

$$m_i^L = \lfloor m_i \rfloor$$

$$m_i^H = \lceil m_i \rceil$$

$$Q_{es}^{(i)} = \frac{es_{m_i^L} + es_{m_i^H}}{2}$$ ,

where $\lfloor m_i \rfloor$ and $\lceil m_i \rceil$ are values obtained by rounding down and up the fractional part of $m_i$, respectively. For the sake of convenience, $Q_{es}^{(0)}$ and $Q_{es}^{(Q_{es})}$ are defined by:

$$Q_{es}^{(0)} = es_1$$

$$Q_{es}^{(Q_{es})} = es_p$$ .

(5) Division procedure (2): calculation of $Q_{RAF}$ quantiles for RAF

For a positive integer $Q_{RAF}$, ($Q_{RAF}$ - 1) values dividing the distribution into $Q_{RAF}$ equal parts are computed. A specific method of calculating quantiles is shown below, but the method of calculating the quantiles is not limited thereto. When the data obtained by sorting RAFs of the SNPs in ascending order is $RAF_1 \le RAF_2 \le ... \le RAF_p$, the *j*-th $Q_{RAF}$-quantile

$Q_{RAF}^{(j)}$ $(1 \le j \le Q_{RAF} - 1)$ is given by:

$$m_j = \frac{j \times p}{Q_{RAF}}$$

$$m_j^L = \lfloor m_j \rfloor$$

$$m_j^H = \lceil m_j \rceil$$

$$Q_{RAF}^{(j)} = \frac{RAF_{m_j^L} + RAF_{m_j^H}}{2}$$ ,

where $\lfloor m_j \rfloor$ and $\lceil m_j \rceil$ are values obtained by rounding down and up the fractional part of $m_j$, respectively. For the sake of convenience, $Q_{RAF}^{(0)}$ and $Q_{RAF}^{(Q_{RAF})}$ are defined by:

$$Q_{RAF}^{(0)} = RAF_1$$

$$Q_{RAF}^{(Q_{RAF})} = RAF_p$$ .

(6) Classification of SNPs

The $p$ SNPs are classified into $Q_{es}$-by-$Q_{RAF}$ categories using the results of $Q_{es}^{(i)} \ (0 \leq i \leq Q_{es})$ and $Q_{RAF}$-quantiles $Q_{RAF}^{(j)} \ (0 \leq j \leq Q_{RAF})$ calculated by the aforementioned process. When the effect size and RAF of the $k$-th SNP ($1 \leq k \leq p$) is $es_k$ and $RAF_k$, respectively, a category $cat_k$ of the $k$-th SNP is defined by:

$$cat_k = (i^k, j^k)$$

$$s.t. Q_{es}^{(i^k-1)} \leq es_k \leq Q_{es}^{(i^k-1)}, \quad Q_{RAF}^{(j^k-1)} \leq RAF_k \leq Q_{RAF}^{(j^k-1)}$$ .

(7) Estimation of parameters of genetic architecture

Parameters of genetic architecture such as the effect size and RAF can be estimated by association analysis of polymorphisms with traits. For the analysis of association between of polymorphisms and traits, a program available to the public can be used, and for example, PLINK or GCTA available on the Internet may be used.

(8) Calculation of genomic similarity matrix

The "genomic similarity matrix" refers to an $N$-by-$N$ matrix representing similarities between individuals based on genomic information. Here, the genomic similarity matrix is calculated for each of the $Q_{es}$-by-$Q_{RAF}$ categories. A typical equation for calculating a genomic similarity matrix $A$ is shown below, but equations for calculating genomic similarity matrices are not limited thereto:

$$A^{(i,j)} = \frac{1}{p^{(i,j)}} W^{(i,j)} W^{(i,j)\prime}$$

,

where $A^{(i,j)}$ is a genomic similarity matrix *(N by N* dimensions) for the category *(i,j)*, $p^{(i,j)}$ is the number of SNPs belonging to the category *(i,j)*, $W^{(i,j)}$ is a submatrix *(N by* $p^{(i,j)}$ dimensions) obtained by taking a column vector or vectors of SNPs belonging to the category *(i,j)* from the matrix *W,* and $W^{(i,j)\prime}$ is a transpose of the submatrix $W^{(i,j)}$.

(9) Use of linear mixed models

(9-1) Use of genetic architectures

As a prediction model using genomic information, a linear mixed model is given by:

$$y = \mu \mathbf{1}_N + g + \varepsilon$$

$$g = \sum_{i,j} g^{(i,j)}$$

$$g^{(i,j)} \sim N(\mathbf{0}, \sigma_g^{2(i,j)} A^{(i,j)})$$

$$\varepsilon \sim N(\mathbf{0}, \sigma_e^2 I)$$

,

where $y$ is a vector *(N* dimension) of traits, $\mu$ is a mean value of traits, $\mathbf{1}_N$ is a column vector *(N* dimension) of which elements are all 1, $g$ is a vector *(N* dimension) of genetic contributions to a trait, $\varepsilon$ is a residual vector *(N* dimension), $g^{(i,j)}$ is a vector *(N* dimension) of contributions of SNPs belonging to the category *(i,j)* to a trait, $A^{(i,j)}$ is a genomic similarity matrix *(N* by *N* dimensions) for the category *(i,j)*, *I* is an identity matrix *(N* by *N* dimensions), $N(0, \sigma_g^{2(i,j)} A^{(i,j)})$ represents a multivariate normal distribution (with mean vector 0 and variance-covariance structure $\sigma_g^{2(i,j)} A^{(i,j)}$), and $N(0, \sigma_e^2 I)$ represents a multivariate normal distribution (with mean vector 0 and variance-covariance structure $\sigma_e^2 I$).

(9-2) With gender/age information

As a prediction model using genomic information and gender/age information, a linear mixed model is given by:

$$y = \mu \mathbf{1}_N + X\beta + g + \varepsilon$$

$$g \sim N(\mathbf{0}, \sigma_g^2 A)$$

$$\varepsilon \sim N(\mathbf{0}, \sigma_e^2 I)$$

,

where $y$ is a vector *(N* dimension) of traits, $\mu$ is a mean value of traits, $\mathbf{1}_N$ is a column vector *(N* dimension) of which elements are all 1, $X$ is a matrix *(N* by 6 dimensions) containing the gender/age information, $\beta$ is a weight for gender or age variables (6 *dimension)*, $g$ is a vector *(N* dimension) of genetic contributions to a trait, $\varepsilon$ is a residual vector *(N* dimension), $A$ is a genomic similarity matrix *(N* by *N* dimensions) when $Q_{es} = 1$ and $Q_{RAF} = 1$, *I* is an identity matrix *(N* by *N* dimensions), $N(0, \sigma_g^2 A)$ represents a multivariate normal distribution (with mean vector 0 and variance-covariance

structure $\sigma_g^2 A$), and $N(0, \sigma_e^2 I)$ represents a multivariate normal distribution (with mean vector 0 and variance-covariance structure $\sigma_e^2 I$).

(9-3) With genetic architectures and gender/age information

As a prediction model using genomic information and gender/age information, a linear mixed model is given by:

$$y = \mu 1_N + X\beta + g + \varepsilon$$

$$g = \sum_{i,j} g^{(i,j)}$$

$$g^{(i,j)} \sim N(0, \sigma_g^{2(i,j)} A^{(i,j)})$$

$$\varepsilon \sim N(0, \sigma_e^2 I)$$

,

where $y$ is a vector *(N dimension)* of traits, $\mu$ is a mean value of traits, $1_N$ is a column vector *(N dimension)* of which elements are all 1, $X$ is a matrix *(N by 6 dimensions)* containing the gender/age information, $\beta$ is a weight for gender or age variables (6 *dimension*), $g$ is a vector *(N dimension)* of genetic contributions to a trait, $\varepsilon$ is an residual vector *(N dimension)*, $g^{(i,j)}$ is a vector *(N dimension)* of contributions of SNPs belonging to the category *(i,j)* to a trait, $A^{(i,j)}$ is a genomic similarity matrix *(N by N dimensions)* for the category *(i,j)*, $I$ is an identity matrix *(N by N dimensions)*, $N(0, \sigma_g^{2(i,j)} A^{(i,j)})$ represents a multivariate normal distribution (with mean vector 0 and variance-covariance structure $\sigma_g^{2(i,j)} A^{(i,j)}$), and $N(0, \sigma_e^2 I)$ represents a multivariate normal distribution (with mean vector 0 and variance-covariance structure $\sigma_e^2 I$).

(10) Estimation of parameters in linear mixed models

Parameters ($\mu, \beta, \sigma_g^{2(i,j)}, \sigma_e^2$) in linear mixed models can be estimated using the restricted maximum likelihood (REML) approach. For REML, a commonly available program can be used, and GCTA which can be downloaded free of charge from the Internet or a commercial program ASReml may be used. Average Information REML, Fisher-scoring REML, and EM can be used for estimation of parameters in the GCTA and Average Information REML can be used for estimation of parameters in the ASReml. Hereinafter, the estimated parameters are denoted as $\tilde{\mu}, \hat{\beta}, \widehat{\sigma_g^2}^{(i,j)}$, and $\widehat{\sigma_e^2}$.

(11) Estimation of contribution ratio

A contribution ratio $V_G^{(i,j)}/V_P$ for the SNPs belonging to the category *(i,j)* is defined by the following equation using the parameters $(\widehat{\sigma_g^2}^{(i,j)}, \widehat{\sigma_e^2})$ estimated by REML:

$$V_G^{(i,j)}/V_P = \frac{\widehat{\sigma_g^2}^{(i,j)}}{\widehat{\sigma_g^2}^{(i,j)} + \sigma_e^2}$$

.

The total contribution ratio $V_G/V_P$ for all SNPs is defined by:

$$V_G/V_P = \sum_{i,j} V_G^{(i,j)}/V_P$$
.

(12) Prediction of contributions by genetic factors

Hidden variables *(g, g^(i,j), ε)* of the linear mixed model are not included in the REML likelihood function and thus cannot be estimated, but they can be predicted by:

$$\widehat{g}^{(i,j)} = \widehat{\sigma_g^2}^{(i,j)} A^{(i,j)} P y$$

$$\widehat{g} = \sum_{ij} \widehat{g}^{(i,j)}$$

$$\hat{\epsilon} = y - \widehat{g}$$
,

where P is an *N*-by-*N* matrix given by P = $V^{-1}$ - $V^{-1}$ $\dot{X}(\dot{X}'V^{-1} \dot{X})^{-1}$ $\dot{X}'V^{-1}$, *V* is an *N*-by-*N* matrix given by $V = \sum_{i,j} \widehat{\sigma_g^2}^{(i,j)} A^{(i,j)} + \widehat{\sigma_e^2} I$, *y* is a vector (*N* dimension) of traits, and $\dot{X}$ is an *N*-by-7 matrix given by $\dot{X} = (1_N, X)$. Hereinafter, the predicted hidden variables are denoted as $\hat{g}$, $\hat{g}^{(i,j)}$, and $\hat{\epsilon}$.

(13) Trait prediction

When the estimated parameters ($\hat{\mu}_t$, $\hat{\beta}_t$, $\widehat{\sigma_g^2}_t^{(i,j)}$, $\widehat{\sigma_e^2}_t$) and predicted hidden variables ($\hat{g}_t^{(i,j)}$, $\hat{\epsilon}_t$) have been obtained using the aforementioned method from a set of training data ($y_t$, $X_t$, $W_t$) for $N_t$ individuals with all of the genomic information, gender/age information, and phenotypic information and genomic information ($W_v$) and gender/age information ($X_v$) for $N_v$ individuals to be predicted have been obtained but phenotypic information ($y_v$) is unknown, a predicted value $\hat{y}_v$ (*N* dimension) of the unknown phenotypic information can be given by:

$$\hat{u}_t^{(i,j)} = \frac{1}{N} W_t^{(i,j)'} A_t^{(i,j)^{-1}} \hat{g}_t^{(i,j)}$$

$$\hat{y}_v = \hat{\mu}_t 1_{N_v} + X_v \widehat{\beta}_t + \sum_{i,j} W_v^{(i,j)} \hat{u}_t^{(i,j)} \qquad (1)$$

where $W_t^{(i,j)}$ is a submatrix ($N_t$ by $p^{(i,j)}$ dimensions) obtained by taking a column vector or vectors of SNPs belonging to the category (*i,j*) from the matrix $W_t$, $A^{(i,j)}$ is a genomic similarity matrix ($N_t$ by $N_t$ dimensions) calculated from $W_t^{(i,j)}$, $\hat{g}_t^{(i,j)}$ is an predicted hidden variable ($N_t$ dimension) calculated from a set of training data, $\hat{\mu}_t$ is a mean value of traits, $1_{Nv}$ is a column vector ($N_v$ dimension) of which elements are all 1, $\hat{\mu}_t^{(i,j)}$ is a weight vector ($p^{(i,j)}$ dimension) for each SNP belonging to the category (*i,j*) calculated from a set of training data, and $W_v^{(i,j)}$ is a submatrix ($N_v$ by $p^{(i,j)}$ dimensions) obtained by taking a column vector or vectors of SNPs belonging to the category (*i,j*) from a genomic information matrix $W_v$ for a set of data to be predicted.

As a special example of Equation (1), the following Equations (2) and (3) can be considered:

$$\widehat{\boldsymbol{y}}_v = \hat{\mu}_t \mathbf{1}_{N_v} + \boldsymbol{X}_v \widehat{\boldsymbol{\beta}}_t \tag{2}$$

$$\widehat{\boldsymbol{y}}_v = \hat{\mu}_t \mathbf{1}_{N_v} + \sum_{i,j} \boldsymbol{W}_v^{(i,j)} \widehat{\boldsymbol{u}}_t^{(i,j)} \tag{3}$$

Equation (2) represents a equation for predicting traits using only the gender/age information, and Equation (3) represents a equation for predicting traits using only the genomic information. Furthermore, when $Q_{es}$ = 1 and $Q_{RAF}$ = 1, then the following Equations (4) and (5) can be considered as special cases of Equations (1) and (3), respectively:

$$\widehat{\boldsymbol{y}}_v = \hat{\mu}_t \mathbf{1}_{N_v} + \boldsymbol{X}_v \widehat{\boldsymbol{\beta}}_t + \boldsymbol{W}_v^{(1,1)} \widehat{\boldsymbol{u}}_t^{(1,1)} \tag{4}$$

$$\widehat{\boldsymbol{y}}_v = \hat{\mu}_t \mathbf{1}_{N_v} + \boldsymbol{W}_v^{(1,1)} \widehat{\boldsymbol{u}}_t^{(1,1)} \tag{5}$$

Equation (1) is designated as a "genetic architecture division + gender/age adjustment method," Equation (2) is designated as a "gender/age adjustment method," Equation (3) is designated as a "genetic architecture division method," Equation (4) is designated as a "genetic architecture non-division + gender/age adjustment method," and Equation (5) is designated as a "genetic architecture non-division method."

(14) Trait prediction system

In order to automate the aforementioned methods of predicting traits, they can be programmed so that they can be executed by a computer. A program thus created is also within the scope of the present invention.
Furthermore, a trait prediction system can be provided which has, in addition to the computer for executing the program, an input device for inputting information such as single nucleotide polymorphism, gender, and age and an output device for outputting results obtained by the execution of the program.

Examples

Single nucleotide polymorphism information of the examples described below was measured using HumanOmniExpressExome chip (Illumina).

Example 1

(Method)

In this example, body heights were focused as an example of a multifactorial quantitative trait. Single nucleotide polymorphism data and gender/age information collected from 4,992 individuals from April 2015 to March 2016 by the Tohoku Medical Megabank Project were used and trait prediction models were made by the method of creating a trait prediction model of the present invention (using the aforementioned (9-2) with gender/age information) to estimate heritability. Heritability was also estimated as controls for cases where no gender/age information was used and compared with those in the cases where the information was used.
Next, the accuracy of prediction by the trait prediction model was evaluated for each of the cases where (1) only the gender/age information was used; (2) only the single nucleotide polymorphism information was used; and (3) both were used (i.e., the examples of the present invention), using a 2-fold cross validation method. The coefficient of determination $R^2$ (i.e., a squared correlation coefficient) between the measured value and the predicted value was used as an evaluation measure.

(Estimation method of heritability)

When $Q_{es}$ = 1 and $Q_{RAF}$ = 1, the proportion of trait variance explained by genetic factors is referred to as heritability $h^2$. A heritability $\widehat{h^2}$ is calculated by the following equation using the parameters $(\widehat{\sigma_g^2}^{(1,1)}, \widehat{\sigma_e^2})$ estimated

by REML:

$$\widehat{h^2} = \frac{\widehat{\sigma_g^2}^{(1,1)}}{\widehat{\sigma_g^2}^{(1,1)} + \widehat{\sigma_e^2}}$$

.

(Results)

The heritability obtained without using the gender/age information was 40.67% whereas the heritability obtained with using the gender/age information was 82.29%. The heritability was significantly increased when the gender/age information was used as compared with the case without using the gender/age information. It was found that a part of the variance of the body height can be accounted for by the gender and age.

The accuracies of prediction ($R^2$) were evaluated for the three cases (1) to (3) using the 2-fold cross validation method (mean $\pm$ standard deviation), which were (1) 56.89 $\pm$ 1.36%, (2) 1.45 $\pm$ 0.26%, and (3) 59.63 $\pm$ 1.24%, respectively. When both of the gender/age information and the genome information were used, the accuracy of prediction increased as compared with the case where only the gender/age information was used and the case where only the genome information was used.

Example 2

(Method)

In this example, a disease of diabetes was focused as an example of a multifactorial quantitative trait. Single nucleotide polymorphism data and gender/age information collected from 4,992 individuals from April 2015 to March 2016 by the Tohoku Medical Megabank Project were used and trait prediction models were made by the method of creating a trait prediction model of the present invention (using the aforementioned (9-2) with gender/age information). According to the results of an HbA1c test, an individual was assumed to suffer from diabetes when the level was 6.5 or higher, and assumed not to suffer from diabetes when the level was lower than 6.5. The accuracy of prediction by the trait prediction model was evaluated for each of the cases where (1) only the gender/age information was used; (2) only the single nucleotide polymorphism information was used; and (3) both were performed (i.e., the examples of the present invention), using a 2-fold cross validation method. AUC was used as an evaluation measure.

(Results)

The accuracies of prediction were (1) 61.39 $\pm$ 1.56%, (2) 55.76 $\pm$ 0.28%, and (3) 62.98 $\pm$ 0.61%. When both of the gender/age information and the genome information were used, the accuracy of prediction increased as compared with the case where only the gender/age information was used and the case where only the genome information was used.

Example 3

(Method)

In this example, HbA1c levels and body heights were focused as examples of a multifactorial quantitative trait. Single nucleotide polymorphism data collected from 4,992 individuals from April 2015 to March 2016 by the Tohoku Medical Megabank Project were used to estimate contribution ratios by the genetic architecture division method. Estimation was performed for two cases: (1) when $Q_{es}$ = 50 and $Q_{RAF}$ = 1, and (2) when $Q_{es}$ = 1 and $Q_{RAF}$ = 30.

(Results)

(1) Fig. 1 shows estimated contribution ratios with $Q_{es}$ = 50 and $Q_{RAF}$ = 1. It was estimated that the contribution ratios for single nucleotide polymorphisms with moderate effect sizes are larger and the contribution ratios for single nucleotide polymorphisms with small effect sizes are extremely small both in the case using the HbA1c levels and the case using the body heights. It was also estimated that the contributions of the single nucleotide polymorphisms with larger effect sizes are large in the case using the HbA1c levels, but the contributions of the single nucleotide polymorphisms with large effect sizes are limited in the case using the body heights.

(2) Fig. 2 shows estimated contribution ratios with $Q_{es}$ = 1 and $Q_{RAF}$ = 30. It was estimated that the contribution ratios for single nucleotide polymorphisms which are not rare are limited and the contribution ratios for single nucleotide polymorphisms which are rare are extremely high in the case using the HbA1c levels. It was also estimated that the contributions of the single nucleotide polymorphisms which are rare are not small but the contributions of the single nucleotide polymorphisms which are not rare are also not small in the case using the body heights.

Example 4

(Method)

In order to show that genetic architecture division method can improve the accuracy of trait prediction when trained with sufficient amount of samples, single nucleotide polymorphism data and HbA1c levels collected from 4,992 individuals from April 2015 to March 2016 by the Tohoku Medical Megabank Project were used. Estimation of effect sizes and allele frequencies as well as estimation of linear mixed models were performed using a set of verification data. Prediction of contribution ratio by genetic factors and calculation of weights to single nucleotide polymorphisms were performed using a set of training data. The accuracy of prediction was verified using a set of verification data. It is thus possible to evaluate the accuracy of prediction for cases where the sample size is sufficiently large.

The accuracies of prediction by the trait prediction models were evaluated for each of the cases with (1) $Q_{es}$ = 1 and $Q_{RAF}$ = 1 (without the genetic architecture division) and (2) $Q_{es}$ = 10 and $Q_{RAF}$ = 1 (with the genetic architecture division; the examples of the present invention), using the 2-fold cross validation method. The coefficient of determination $R^2$ (i.e., a squared correlation coefficient) between the measured value and the predicted value was used as an evaluation measure.

(Results)

The accuracies of prediction were (1) 4.52 $\pm$ 0.16% and (2) 16.52 $\pm$ 0.30%. It was demonstrated that the accuracy of prediction can remarkably be improved with the genetic architecture division as compared with the cases without the genetic architecture division.

Example 5

(Method)

In this example, for 27 quantitative traits and 5 qualitative traits shown in Fig. 3, single nucleotide polymorphism data collected from 4,992 individuals from April 2015 to March 2016 by the Tohoku Medical Megabank Project were used and trait prediction models were made by the method of creating a trait prediction model of the present invention (using the aforementioned (9-3) with genetic architectures and gender/age information). The accuracy of prediction by the trait prediction model was evaluated for each of the cases where (1) only the single nucleotide polymorphism information was used and $Q_{es}$ = 1 and $Q_{RAF}$ = 1 (without the genetic architecture division); (2) only the gender/age information was used; and (3) both the single nucleotide polymorphism information and the gender/age information were used and $Q_{es}$ = 1 and $Q_{RAF}$ = 1 (without the genetic architecture division; the examples of the present invention), using a 2-fold cross validation method. The coefficient of determination $R^2$ (i.e., a squared correlation coefficient) between the measured value and the predicted value was used as an evaluation measure for the quantitative data and AUC was used for the qualitative data.

(Results)

Figs. 4 and 5 show the results of accuracy evaluation for the 27 quantitative traits and 5 qualitative traits, respectively. For all of the 27 quantitative traits and the 5 qualitative traits shown in Figs. 4 and 5, it was demonstrated that the accuracies of prediction in (3) both the single nucleotide polymorphism information and the gender/age information were used and $Q_{es}$ = 1 and $Q_{RAF}$ = 1 (without the genetic architecture division; the examples of the present invention) were higher than in (1) only the single nucleotide polymorphism information was used and $Q_{es}$ = 1 and $Q_{RAF}$ = 1 (without the genetic architecture division); (2) only the gender/age information was used.

Example 6

(Method)

In order to show that the accuracy of trait prediction can be improved by using the gender/age information or both of the single nucleotide polymorphism information and the gender/age information when the training was performed using a sufficient amount of samples. For 27 quantitative traits and 5 qualitative traits shown in Fig. 3, single nucleotide polymorphism data collected from 4,992 individuals from April 2015 to March 2016 by the Tohoku Medical Megabank Project were used and trait prediction models were made by the method of creating a trait prediction model of the present invention (using the aforementioned (9-3) with genetic architectures and gender/age information). The accuracy of prediction by the trait prediction model was evaluated for each of the cases where (1) only the single nucleotide polymorphism information was used and $Q_{es} = 1$ and $Q_{RAF} = 1$ (without the genetic architecture division); (2) only the gender/age information was used; (3) both the single nucleotide polymorphism information and the gender/age information were used and $Q_{es} = 1$ and $Q_{RAF} = 1$ (without the genetic architecture division; the examples of the present invention); and (4) both the single nucleotide polymorphism information and the gender/age information were used and $Q_{es} = 10$ and $Q_{RAF} = 1$ (with the genetic architecture division; the examples of the present invention), using a 2-fold cross validation method. The coefficient of determination $R^2$ (i.e., a squared correlation coefficient) between the measured value and the predicted value was used as an evaluation measure for the quantitative data and AUC was used for the qualitative data. Estimation of effect sizes and allele frequencies as well as estimation of linear mixed models were performed using a set of verification data. Prediction of contribution ratio by genetic factors and calculation of weights to single nucleotide polymorphisms were performed using a set of training data. The accuracy of prediction was verified using a set of verification data.

(Results)

Figs. 6 and 7 show the results of accuracy evaluation for the 27 quantitative traits and 5 qualitative traits, respectively. For all of the 27 quantitative traits and the 5 qualitative traits shown in Figs. 6 and 7, it was demonstrated that the accuracies of prediction in (3) both the single nucleotide polymorphism information and the gender/age information were used and $Q_{es} = 1$ and $Q_{RAF} = 1$ (without the genetic architecture division; the examples of the present invention) were higher than in (1) only the single nucleotide polymorphism information was used and $Q_{es} = 1$ and $Q_{RAF} = 1$ (without the genetic architecture division); (2) only the gender/age information was used. For all traits, the accuracies of prediction in (4) both the single nucleotide polymorphism information and the gender/age information were used and $Q_{es} = 10$ and $Q_{RAF} = 1$ (with the genetic architecture division; the examples of the present invention) were higher, when (3) both the single nucleotide polymorphism information and the gender/age information were used and $Q_{es} = 1$ and $Q_{RAF} = 1$ (without the genetic architecture division; the examples of the present invention) and (4) both the single nucleotide polymorphism information and the gender/age information were used and $Q_{es} = 10$ and $Q_{RAF} = 1$ (with the genetic architecture division; the examples of the present invention) were compared.

(Conclusion)

As shown above, by using a trait prediction model created by a method of creating a trait prediction model of the present invention, traits can be predicted with a higher accuracy than with a conventional prediction method. Furthermore, it is possible to elucidate the genetic architecture of a trait by estimating the contribution ratio by the genetic architecture division method.

Industrial Applicability

According to the present invention, it becomes possible to provide methods of creating a trait prediction model for predicting phenotypic traits from single nucleotide polymorphism data, and methods of predicting traits with which traits can be predicted with a high accuracy.

**Claims**

1.  A method of creating a trait prediction model for predicting a phenotype of a multifactorial trait using data of a plurality of single nucleotide polymorphisms linked to a trait for each of a plurality of individuals of an organism, the method comprising the steps of:

representing each of the plurality of single nucleotide polymorphisms as a matrix;

classifying the plurality of single nucleotide polymorphisms into a plurality of categories based on their genetic architecture;

calculating, for each of the categories, a genomic similarity matrix using the represented matrix and the number of the single nucleotide polymorphisms belonging to the category; and

applying the genomic similarity matrix and a parameter of the genetic architecture to a linear mixed model.

2. The method of creating a trait prediction model according to Claim 1, wherein the genetic architecture is an effect size and/or an allele frequency.

3. A method of creating a trait prediction model for predicting a phenotype of a multifactorial trait using data of gender, age and a plurality of single nucleotide polymorphisms linked to a trait for each of a plurality of individuals of an organism, the method comprising the steps of:

representing each of the plurality of single nucleotide polymorphisms as a matrix;

representing the gender and/or age as a matrix;

calculating a genomic similarity matrix using the represented matrix of the single nucleotide polymorphisms and the number of the single nucleotide polymorphisms; and

applying the genomic similarity matrix and the matrix of the gender and/or age to a linear mixed model.

4. The method of creating a trait prediction model according to Claim 3, wherein the trait is selected from the group consisting of the body height, body weight, systolic blood pressure, diastolic blood pressure, blood glucose, HbA1c, red blood cell number, hemoglobin, corpuscular volume, white blood cell number, platelet number, percentage of neutrophils, percentage of lymphocytes, percentage of monocytes, percentage of eosinophils, percentage of basophils, percentage of large unstained cells, AST (GOT), ALT (GPT), $\gamma$-GTP, total cholesterol, neutral fat, HDL cholesterol, LDL cholesterol, creatinine, urea nitrogen, uric acid, diabetes, hypertension, high LDL cholesterolemia, low HDL cholesterolemia, and hypertriglyceridemia.

5. A method of predicting a trait of an individual of an organism from a plurality of single nucleotide polymorphism data in the individual of the organism, comprising the steps of:

creating a trait prediction model using a set of training data according to the method of creating a trait prediction model according to any one of Claims 1 to 4;

determining a parameter and a hidden variable of a linear mixed model; and

applying the plurality of single nucleotide polymorphism data of the individual of the organism to the trait prediction model.

6. A program for predicting a trait of an individual of an organism from a plurality of single nucleotide polymorphism data in the individual of the organism, wherein the program causes the computer to execute the method according to any one of Claims 1 to 5.

7. A computer readable recording medium in which the program according to Claim 6 has been recorded.

8. A trait prediction system for predicting a trait of an individual of an organism from a plurality of single nucleotide polymorphism data, comprising:

(i) an input device for inputting a plurality of single nucleotide polymorphism data of the individual of the organism;

(ii) a computer that executes the program according to Claim 7 using the input data, and

(iii) an output device for outputting the result obtained in (ii).

FIG. 1

FIG. 2

| Quantitative cohort data | Significant figure |
|---|---|
| Body height | to the tenths place |
| Body weight | to the tenths place |
| Systolic blood pressure | to the ones place |
| Diastolic blood pressure | to the ones place |
| Blood glucose | to the ones place |
| HbA1c | to the tenths place |
| Red blood cell count | to the ones place |
| Hemoglobin | to the tenths place |
| Corpuscular volume | to the tenths place |
| White blood cell count | to the ones place |
| Platelet count | to the tenths place |
| Percentage of neutrophils | to the tenths place |
| Percentage of lymphocytes | to the tenths place |
| Percentage of monocytes | to the tenths place |
| Percentage of eosinophils | to the tenths place |
| Percentage of basophils | to the tenths place |
| Percentage of large unstained cells | to the tenths place |
| AST (GOT) | to the ones place |
| ALT (GPT) | to the ones place |
| $\gamma$-GTP | to the ones place |
| Total cholesterol | to the ones place |
| Neutral fat | to the ones place |
| HDL cholesterol | to the ones place |
| LDL cholesterol | to the ones place |
| Creatinine | to the hundredths place |
| Urea nitrogen | to the ones place |
| Uric acid | to the tenths place |

| Quantitative cohort data | Number of levels |
|---|---|
| Suffering from hypertension | 2 levels |
| Suffering from diabetes | 2 levels |
| Suffering from high LDL cholesterolemia | 2 levels |
| Suffering from low HDL cholesterolemia | 2 levels |
| Suffering from hypertriglyceridemia | 2 levels |

FIG. 3

FIG. 4

FIG. 5

FIG. 6

FIG. 7

**INTERNATIONAL SEARCH REPORT**

| International application No. |
| --- |
| PCT/JP2015/083068 |

A. CLASSIFICATION OF SUBJECT MATTER
*G06F19/18*(2011.01)i

According to International Patent Classification (IPC) or to both national classification and IPC

B. FIELDS SEARCHED

Minimum documentation searched (classification system followed by classification symbols)
G06F19/18

Documentation searched other than minimum documentation to the extent that such documents are included in the fields searched
Jitsuyo Shinan Koho     1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
CiNii, nature.com, SCIENCE

C. DOCUMENTS CONSIDERED TO BE RELEVANT

| Category* | Citation of document, with indication, where appropriate, of the relevant passages | Relevant to claim No. |
| --- | --- | --- |
| A | Kenkyu Daimoku: Cohort Renkei Togo DB ni Motomerareru Genome Joho no Data Ryo no Mitsumori, [online], Iwate Medical University Iwate Tohoku Medical Megabank Organization, 17 June 2014 (17.06.2014), [retrieval date: 28 January 2016 (28.01.2016)], Internet, <URL: http://iwate-megabank.org/wp-content/uploads/2014/01/2014-0003.pdf>, <reference URL: http://iwate-megabank.org/about/publication/> | 1–8 |

☒ Further documents are listed in the continuation of Box C.    ☐ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 28 January 2016 (28.01.16) | 09 February 2016 (09.02.16) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2015/083068

C (Continuation).   DOCUMENTS CONSIDERED TO BE RELEVANT

| Category* | Citation of document, with indication, where appropriate, of the relevant passages | Relevant to claim No. |
|---|---|---|
| A | Kenkyu Daimoku: Order-Made Iryo no Jitsugen Program to Tohoku Medical Megabank Keikaku Nippon Tashisetsu Kyodo Kenkyu Cohort Kenkyu Tamokuteki Cohort ga Renkei shite Jisshi suru Genome Kaiseki, [online], Iwate Medical University Iwate Tohoku Medical Megabank Organization, 15 January 2014 (15.01.2014), [retrieval date: 28 January 2016 (28.01.2016)], Internet, <URL: http://iwate-megabank.org/wp-content/uploads/2014/01/2013-0002.pdf>, <reference URL: http://iwate-megabank.org/about/publication/> | 1-8 |
| A | JP 2013-175135 A  (NTT Data Corp.), 05 September 2013 (05.09.2013), entire text; all drawings (Family: none) | 1-8 |
| A | JP 2008-152592 A  (Hitachi, Ltd.), 03 July 2008 (03.07.2008), entire text; all drawings (Family: none) | 1-8 |
| A | WO 2003/048999 A2  (DNAPRINT GENOMICS, INC.), 12 June 2003 (12.06.2003), entire text; all drawings & JP 2006-503346 A       & US 2007/0042362 A1 & EP 1451754 A2         & CA 2468570 A1 & AU 2002352985 A | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014238252 A

**Non-patent literature cited in the description**

- **V. LYSSENKO et al.** *N Engl J Med,* 2008, vol. 359, 2220-2232
- **S. RIPATTHI et al.** *Lanet,* 2010, vol. 376, 1393-1400
- **C. A. IBRAHIM-VERBAAS et al.** *Stroke,* 2014, vol. 45, 403-412
- **D. SPEED ; D. J. BALDING.** *Genome Research,* 2015, vol. 24, 1550-1557